# EUROPEAN PATENT APPLICATION

(11) **EP 3 112 371 A1**
(43) Date of publication of application: **04.01.2017**
(21) Application number: 15755367.8
(22) Date of filing: 23.02.2015
(51) Int. Cl.: C07D 513/04, A61K 31/444, A61P 7/02, A61P 43/00

(54) **HIGH-PURITY CRYSTALS OF ACTIVE BLOOD COAGULATION FACTOR X (FXA) INHIBITOR**

(30) Priority: 25.02.2014 JP 2014033556
(71) Applicant: Daiichi Sankyo Company, Limited, Tokyo 103-8426 (JP)
(72) Inventor: KAWANAMI, Koutarou, Hiratsuka-shi Kanagawa 254-0014 (JP); KITANI, Yasuo, Hiratsuka-shi Kanagawa 254-0014 (JP)
(74) Representative: Nieuwenhuys, William Francis
(86) International application number: PCT/JP2015/054943
(87) International publication number: WO 2015/129603

(57) **Abstract**

An object of the present invention is to provide a high-purity crystal of a compound represented by formula (1a) that is an activated blood coagulation factor X (FXa) inhibitor. Solution: High-purity crystals of a compound represented by the following formula (1a): 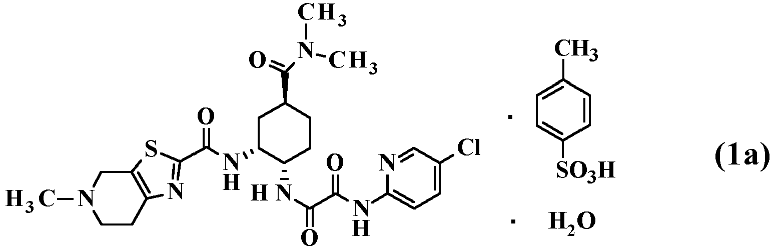 wherein, with regard to the content of impurities, the maximum content of any one type of impurity is 0.03% or less, and the total content of impurities is 0.13% or less, and wherein the high-purity crystals are obtained by a step of dissolving crystals in a solvent and then recrystallizing them.

## Description

### Technical Field

The present invention relates to a method for producing high-purity crystals of a compound that exhibits an inhibitory effect on activated blood coagulation factor X (FXa) and is useful as a prophylactic and/or therapeutic drug for thrombotic diseases.

### Background Art

N¹-(5-Chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate represented by the following formula (1a) (hereinafter, also referred to as compound (1a)): 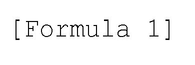 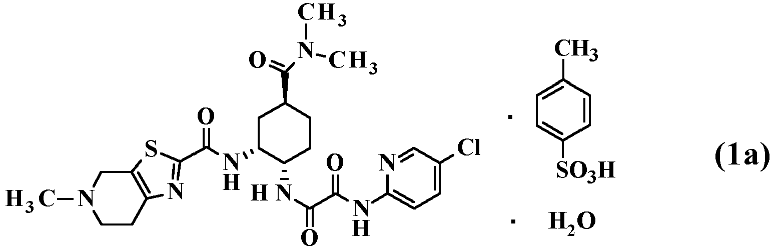 is known as a compound that exhibits an inhibitory effect on activated blood coagulation factor X (FXa), and is useful as a prophylactic and/or therapeutic drug for thrombotic diseases (for example, see Patent Literatures 1 to 8).

However, these patent literatures do not describe high-purity crystals of compound (1a).

### Citation List

### Patent Literature

Patent Literature 1: International Publication No. WO 03/000657
Patent Literature 2: International Publication No. WO 03/000680
Patent Literature 3: International Publication No. WO 03/016302
Patent Literature 4: International Publication No. WO 04/058715
Patent Literature 5: International Publication No. WO 05/047296
Patent Literature 6: International Publication No. WO 07/032498
Patent Literature 7: International Publication No. WO 08/129846
Patent Literature 8: International Publication No. WO 08/156159

### Summary of Invention

### Technical Problem

An object of the present invention is to provide a high-purity crystal of compound (1a) that is an activated blood coagulation factor X (FXa) inhibitor.

### Solution to Problem

The present inventors have focused on impurities contained in trace amounts in the known N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (wherein possibly contained impurities are enantiomers, all stereoisomers, byproducts generated in the course of the reaction, and substances derived from production intermediates, such as N-[(1S,2R,4S)-2-amino-4-(N,N-dimethylcarbamoyl)cyclohexyl]-N'-(5-chloropyridin-2-yl)oxamide, tert-butyl [(1R,2S,5S)-2-({[(5-chloropyridin-2-yl)amino](oxo)acetyl}amino)-5-(N,N-dimethylcarbamoyl)cyclohexyl]carbamate, and N,N'-bis(5-chloropyridin-2-yl)ethanediamide), and as a result, the present inventors have succeeded in producing high-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate having a low content of impurities, thereby completing the present invention.

Specifically, the present invention is as described below.
(1) High-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate represented by the following formula (1a): 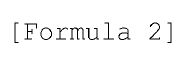 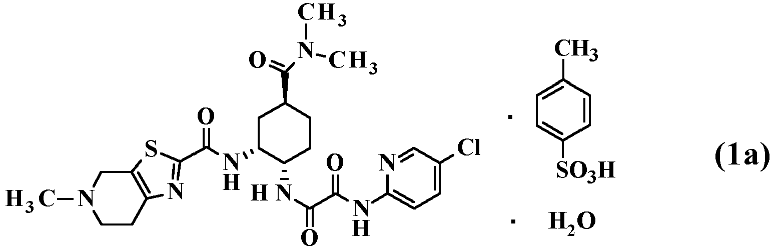 wherein, with regard to the content of impurities, the maximum content of any one type of impurity is 0.03% or less, and the total content of all impurities is 0.13% or less (wherein % by content indicates % with respect to the HPLC area value of a free form of the compound represented by formula (1a)).
(2) High-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate represented by the following formula (1a): 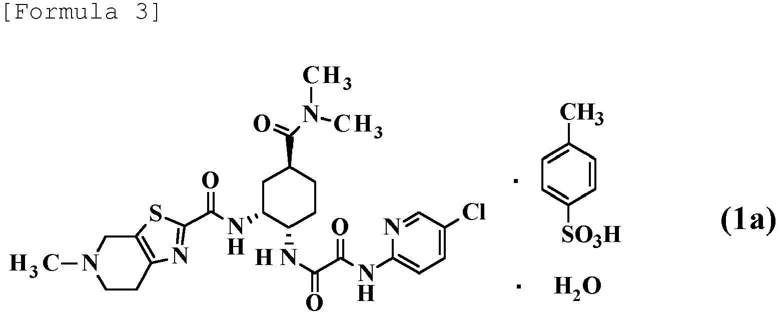
   wherein, with regard to the content of impurities, the maximum content of any one type of impurity is 0.03% or less, and the total content of all impurities is 0.13% or less (wherein % by content indicates % with respect to the HPLC area value of a free form of the compound represented by formula (1a)), and wherein
   the high-purity crystals are obtained by the following step 1 to step 4:
      [Step 1] adding, to N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (compound (1a)), approximately 7 parts by volume of aqueous ethanol [anhydrous ethanol:purified water = 7:3 (V/V)] based on 1 part by weight of compound (1a), and then heating the obtained mixture to an internal temperature of 55°C to 75°C to dissolve compound (1a) in the aqueous ethanol, thereby preparing solution 1,
      [Step 2] washing and separating insoluble matter in solution 1, using approximately 1 part by volume of aqueous ethanol and then using approximately 2 parts by volume of anhydrous ethanol based on 1 part by weight of compound (1a), and thereafter, heating the solution, after completion of the washing and the separation, to an internal temperature of 55°C to 75°C for dissolution, thereby preparing solution 2,
      [Step 3] gradually cooling solution 2 to room temperature, adding, to solution 2, approximately 14 parts by volume of anhydrous ethanol based on 1 part by weight of compound (1a), and then cooling the obtained mixture to an internal temperature of 0°C to 15°C to precipitate crystals, and
      [Step 4] collecting the crystals, washing the collected crystals with approximately 2 parts by volume of anhydrous ethanol based on 1 part by weight of compound (1a), and then drying the crystals under reduced pressure at a temperature of 50°C or lower.
(3) High-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (1a) represented by the following formula (1a): 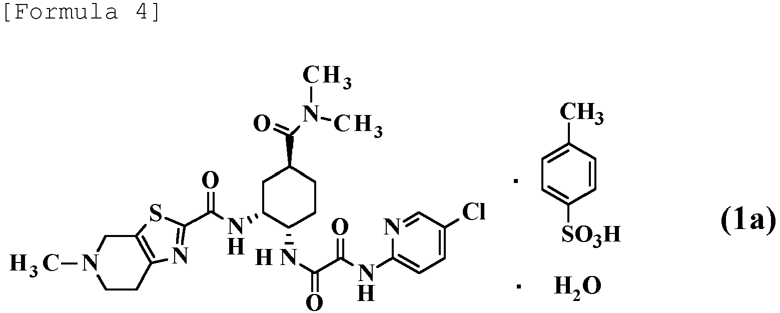
   wherein, with regard to the content of impurities, the maximum content of any one type of impurity is 0.03% or less, and the total content of all impurities is 0.12% or less (wherein % by content indicates % with respect to the HPLC area value of a free form of the compound represented by formula (1a)), and wherein
   the step 1 to the step 4 of (2) above are repeated.
(4) A method for producing high-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate, wherein the high-purity crystals are obtained by the step 1 to the step 4 of (2) above.
(5) Use, as reference standards, of the high-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (1a), which are produced by the production method of (4) above.

### Advantageous Effects of Invention

According to the present invention, high-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate having a low content of impurities can be produced and provided.

According to the present invention, high-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate having a low content of impurities, which can be used as reference standards and the like, can be produced and provided.

### Description of Embodiments

Specifically, the present invention relates to the following:

(N¹-(5-Chloropyridin-2-yl)-N²-((1S,2R,4S)-4-[(dimethylamino)carbonyl]-2-{[(5-methyl-4,5,6,7-tetrahydrothiazolo[5,4-c]pyridine-2-yl)carbonyl]amino}cyclohexyl)ethanediamide) represented by the following formula (1): 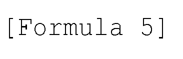 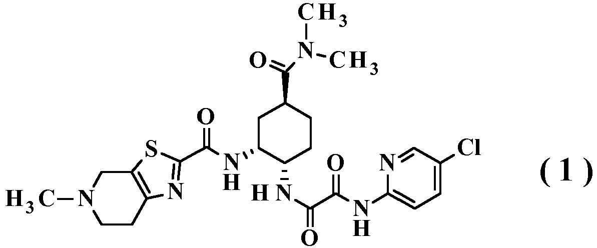 is a free form of a compound (1a), and it has been registered as International Nonproprietary Name (INN): edoxaban, (N-(5-chloropyridin-2-yl)-N'-[(1S,2R,4S)-4-(N,N-dimethylcarbamoyl)-2-(5-methyl-4,5,6,7-tetrahydro[1,3]thiazolo[5,4-c]pyridine-2-carboxamido)cyclohexyl]oxamide) in the World Health Organization (WHO).

The above described compound (1) may be a pharmacologically acceptable salt and/or hydrate thereof, and compound (1) is preferably N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate represented by the following formula (1a):

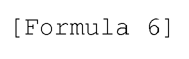

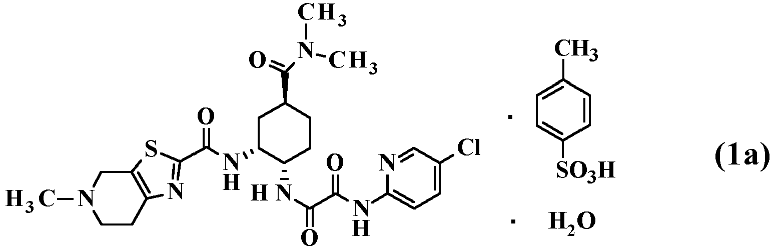

Compound (1a) of the present invention and a method for producing the same are provided by steps 1 to 4 as described below.

As N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate that is a compound (1a) used in step 1, a compound produced by a known production method may be used and, for example, a compound produced by the production method described in International Publication No. WO 07/032498 may be used, as described in the reference example described later.

Hereinafter, [step 1] to [step 4] will be described in detail.

[Step 1] is a step of dissolving compound (1a) in aqueous ethanol to prepare solution 1.

Herein, as aqueous ethanol used in the present invention, aqueous ethanol prepared by mixing anhydrous ethanol and purified water at a volume ratio (V/V) of 7:3 is used.

More specifically, 7 parts by volume of the above described aqueous ethanol based on 1 part by weight of compound (1a) is added to compound (1a), and the internal temperature in the flask vessel is then increased to 55°C to 75°C, so that compound (1a) is dissolved in the aqueous ethanol, thereby preparing solution 1.

[Step 2] is a step of subjecting solution 1 prepared in [step 1] to hot microfiltration, then washing the filter and filter residue with aqueous ethanol, and further with anhydrous ethanol, then heating again the filtrate obtained by combining the filtered solution and the washing solution to the internal temperature in the reaction vessel (55°C to 75°C) for dissolution, and then dissolving partially precipitated crystals in the solution to prepare solution 2.

Herein, the filter paper used in filtration is preferably a filter paper consisting of glass fibers. Such a filter paper consisting of glass fibers may be a commercially available product, and examples of such a commercially available product include GFP-25 and GFP-60 [both of which are manufactured by Kiriyama Glass Co.]. Moreover, filtration may be carried out under any one of reduced pressure, increased pressure, and ordinary pressure. When filtration is carried out under reduced pressure, attention should be paid to the clogging of the filter paper by precipitation of crystals. Furthermore, before or at the same time as the hot microfiltration step, filtration may be carried out using activated carbon, and the activated carbon used can be a commercially available product. The aqueous ethanol used to wash the filter and the filter residue is preferably used in an amount of 1 part by volume based on 1 part by weight of compound (1a).

Further, the anhydrous ethanol used for washing is preferably used in an amount of 2 parts by volume based on 1 part by weight of compound (1a). In order to increase the internal temperature in the reaction vessel to 55°C to 75°C, the temperature applied to the external portion is preferably approximately 75°C. By the operation of such a step 2, solution 2 with an internal temperature of 55°C to 75°C is prepared.

[Step 3] is a step of gradually cooling solution 2 prepared in step 2, so as to complete crystallization.

In step 3, after dissolution has been observed, solution 2 is gradually cooled to an internal temperature of 50°C to 65°C, so that crystallization is initiated. Thereafter, after solution 2 has been cooled to almost room temperature (internal temperature: 35°C or lower), to solution 2 is added 14 parts by volume of anhydrous ethanol based on 1 part by weight of compound (1a) for dilution. After dilution with the ethanol, the reaction solution is cooled to an internal temperature of 0°C to 15°C to complete precipitation of crystals.

[Step 4] is a step of collecting the precipitated crystals, and then washing and drying them.

The crystals are collected by filtration, and are then washed with anhydrous ethanol in an amount of 2 parts by volume based on 1 part by weight of compound (1a). As described above, filtration may be carried out under any one of reduced pressure, increased pressure, and ordinary pressure. Herein, the collected crystals are washed with anhydrous ethanol, and are then dried under reduced pressure at a temperature of 50°C or lower. Herein, an ordinary vacuum pump or the like may be used for reducing the pressure. Drying is terminated when the weight of crystals has become constant.

The present invention relates to production of high-purity crystals of compound (1a) by the operation of the above described [step 1] to [step 4]. Such high-purity crystals of compound (1a) can also be produced by repeatedly carrying out the operations of [step 1] to [step 4].

High-purity crystals of compound (1a) produced by such operations are preferably used as reference standards (i.e., standard samples), etc., when uniformity and/or purity among production batches is observed in the production of compound (1a).

### Examples

Hereinafter, the present invention will be described in detail in the following examples. However, the present invention is not intended to be limited to them.

Tetramethylsilane was used as an internal standard in nuclear magnetic resonance (NMR) spectroscopy. Abbreviations indicating multiplicity are as follows: s = singlet, d = doublet, t = triplet, q = quartet, m = multiplet, and brs = broad singlet.

### < Analysis conditions for HPLC >

Column: CAPCELL PAK C18 MGII (Shiseido Co., Ltd.)
Mobile phase: MeCN:phosphate buffer (pH = 7.0) = 10:90 to 30:70
Temperature: 40°C, Flow rate: 1 mL/min, and Detection wavelength: 290 nm

### (Reference Example 1) Synthesis of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (1a) (the method described in International Publication No. WO 07/032498)

N¹-(5-Chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide (86.8g), which had been produced by the method described in International Publication No. WO 07/032498, was dissolved in 70% aqueous ethanol (418 ml) at 60°C, and thereafter, a solution of p-toluenesulfonic acid monohydrate (29.0g) in 70% aqueous ethanol (167 ml) was added to the above obtained solution. The reaction mixture was stirred at 70°C for 1 hour, and thereafter, the reaction solution was gradually cooled to room temperature. Ethanol was added to the reaction solution, and the obtained mixture was then stirred for 16 hours. Thereafter, the reaction solution was stirred for 1 hour under cooling on ice, and crystals were then collected by filtration to obtain 102.9 g of the title compound.

It was found that the obtained compound had an absorption peak with the same strength at the same wave number as that of a standard preparation (known compound) in the IR.

Moreover, as a result of the analysis of the obtained compound using HPLC, a plurality of impurity peaks (all of which were 0.03% by weight or less) were observed as impurities, and the total content of all impurities was 0.16% by weight. Thus, the purity thereof was found to be 99.84% (wherein % by content indicates % with respect to the HPLC area value of a free form of the compound represented by formula (1a)).

¹H-NMR (DMSO-d6) δ: 1.45-1.54 (1H, m), 1.66-1.78 (3H, m), 2.03-2.10 (2H, m), 2.28 (3H, s), 2.79 (3H, s), 2.91-3.02 (1H, m), 2.93 (3H, s), 2.99 (3H, s), 3.13-3.24 (2H, m), 3.46-3.82 (2H, m), 3.98-4.04 (1H, m), 4.43-4.80 (3H, m), 7.11 (2H, d, J = 7.8 Hz), 7.46 (2H, d, J = 8.2 Hz), 8.01 (2H, d, J = 1.8 Hz), 8.46 (1H, t, J = 1.8 Hz), 8.75 (1H, d, J = 6.9 Hz), 9.10-9.28 (1H, br. s), 10.18 (1H, br. s), 10.29 (1H, s).
Elemental analysis: Anal. Calcd. For: C; 50.43%, H; 5.46%, N; 13.28%.
Found: C; 50.25%, H; 5.36%, N; 13.32%

### (Example 1) N¹-(5-Chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (1a)

Anhydrous ethanol (350mL) was mixed with purified water (150mL) to prepare aqueous ethanol. This aqueous ethanol was used in Examples 1 to 3.

The N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (compound 1a) (10g), which had been produced in Reference Example 1, was placed in a flask, aqueous ethanol (70 mL) was then added to the flask, and the thus obtained mixture was then stirred at an external temperature of 75°C for 20 minutes. Dissolution of the compound was observed when the temperature of the solution in the flask was 65°C. Using a filter paper made of glass fibers, the solution was poured through the filter paper for filtration. The filter and the filter paper were washed with aqueous ethanol (10 mL), which was used in an amount of 1 part by volume based on 1 part by weight of compound (1a), and subsequently, they were washed with anhydrous ethanol (20 mL), which was used in an amount of 2 parts by volume based on 1 part by weight of compound (1a). The filtered solution was combined with the washing solution (wherein precipitation of some crystals was observed when the temperature of the solution was 45°C), and thereafter, the external temperature was increased to 75°C, and the obtained mixture was then stirred. Since dissolution was observed at a time point at which the internal temperature reached 62°C, the external temperature was decreased to the same temperature as that of the internal temperature. Subsequently, the external temperature was gradually decreased to room temperature (approximately 25°C) over 4 hours. The external temperature was further decreased to 6°C, and thus, it took 1.5 hours to decrease the internal temperature to 6°C. Anhydrous ethanol (140 mL) was added to the mixed solution obtained as a result of the stirring, in an amount of 14 parts by volume of the anhydrous ethanol based on 1 part by weight of compound (1a), and the obtained mixture was then stirred for 1 hour. (After addition of the anhydrous ethanol, the internal temperature increased to 15°C. However, the stirring operation was continued for approximately 1 hour, and as a result, the internal temperature became 6°C.) The precipitated crystals were collected by filtration, and the filtered crystals were then washed with anhydrous ethanol (20 mL), which was used in an amount of 2 parts by volume based on 1 part by weight of compound (1a). The crystals (12.33 g) were dried under reduced pressure at room temperature for 1 hour (the weight of the crystals: 9.15 g), and thereafter, the crystals were further dried under reduced pressure for 1 hour to obtain the title product [9.15 g, yield (recovery rate) = 91.5%] in the form of white crystals.

It was found that the obtained compound had an absorption peak with the same strength at the same wave number as that of a standard preparation (known compound) in the IR.

Moreover, as a result of the analysis of the obtained compound using HPLC, a plurality of impurity peaks (all of which were 0.03% by weight) were observed as impurities, and the total content of all impurities was 0.13% by weight. Thus, the purity thereof was found to be 99.87% (wherein % by content indicates % with respect to the HPLC area value of a free form of the compound represented by formula (1a)).

Less than 300 ppm ethanol was found as a residual solvent. Furthermore, as a result of the measurement of heavy metal and ignition residue, it was found that almost no inorganic impurities remained.

### (Example 2) N¹-(5-Chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (1a)

The N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (compound (1a), 8 g), which had been produced in Example 1, was placed in a flask, aqueous ethanol (56 mL) was then added to the flask, and the thus obtained mixture was then stirred at an external temperature of 75°C for 15 minutes. Dissolution of the compound was observed when the temperature of the solution in the flask was 64°C. Using a filter paper made of glass fibers, the solution was poured through the filter paper for filtration. The filter and the filter paper were washed with aqueous ethanol (8 mL), and subsequently, they were washed with anhydrous ethanol (16 mL). The filtered solution was combined with the washing solution (wherein precipitation of some crystals was observed when the temperature of the solution was 40°C), and thereafter, the external temperature was increased to 75°C, and the obtained mixture was then stirred. Since dissolution was observed at a time point at which the internal temperature reached 63°C, the external temperature was decreased to the same temperature as that of the internal temperature. Subsequently, the external temperature was gradually decreased to room temperature (approximately 25°C) over 2.5 hours. The external temperature was further decreased to 6°C, and thus, it took 1.5 hours to decrease the internal temperature to 6°C. Anhydrous ethanol (112 mL) was added to the mixed solution obtained as a result of the stirring, and the obtained mixture was then stirred for 1 hour. (After addition of the anhydrous ethanol, the internal temperature increased to 14°C. However, the stirring operation was continued for approximately 1 hour, and as a result, the internal temperature became 6°C.) The precipitated crystals were collected by filtration, and the filtered crystals were then washed with anhydrous ethanol (16 mL). The crystals (9.02 g) were dried under reduced pressure at room temperature for 1 hour (the weight of the crystals: 7.33 g), and thereafter, the crystals were further dried under reduced pressure for 1 hour to obtain the title product [7.33 g, yield (recovery rate) = 91.6%] in the form of white crystals.

It was found that the obtained compound had an absorption peak with the same strength at the same wave number as that of a standard preparation (known compound) in the IR.

Moreover, as a result of the analysis of the obtained compound using HPLC, a plurality of impurity peaks (all of which were 0.03% by weight) were observed as impurities, and the total content of all impurities was 0.12% by weight. Thus, the purity thereof was found to be 99.88% (wherein % by content indicates % with respect to the HPLC area value of a free form of the compound represented by formula (1a)).

Less than 300 ppm ethanol was found as a residual solvent. Furthermore, as a result of the measurement of heavy metal and ignition residue, it was found that almost no inorganic impurities remained.

### (Example 3) N¹-(5-Chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (1a)

The N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonic acid monohydrate (compound (1a), 6.08g), which had been produced in Example 2, was placed in a flask, aqueous ethanol (42 mL) was then added to the flask, and the thus obtained mixture was then stirred at an external temperature of 75°C for 10 minutes. Dissolution of the compound was observed when the temperature of the solution in the flask was 63°C. Using a filter paper made of glass fibers, the solution was poured through the filter paper for filtration. The filter and the filter paper were washed with aqueous ethanol (6 mL), and subsequently, they were washed with anhydrous ethanol (12 mL). The filtered solution was combined with the washing solution (wherein precipitation of some crystals was observed when the temperature of the solution was 42°C), and thereafter, the external temperature was increased to 75°C, and the obtained mixture was then stirred. Since dissolution was observed at a time point at which the internal temperature reached 63°C, the external temperature was decreased to the same temperature as that of the internal temperature. Subsequently, the external temperature was gradually decreased to room temperature (approximately 25°C) over approximately 4 hours. The external temperature was further decreased to 6°C, and thus, it took approximately 40 minutes to decrease the internal temperature to 6°C. Anhydrous ethanol (84 mL) was added to the mixed solution obtained as a result of the stirring, and the obtained mixture was then stirred for 1 hour. (After addition of the anhydrous ethanol, the internal temperature increased to 15°C. However, the stirring operation was continued for approximately 1 hour, and as a result, the internal temperature became 6°C.) The precipitated crystals were collected by filtration, and the filtered crystals were then washed with anhydrous ethanol (12 mL). The crystals (6.30 g) were dried under reduced pressure at room temperature for 1 hour (the weight of the crystals: 5.49 g), and thereafter, the crystals were further dried under reduced pressure for 1 hour to obtain the title product [5.49 g, yield (recovery rate) = 91.5%] in the form of white crystals.

It was found that the obtained compound had an absorption peak with the same strength at the same wave number as that of a standard preparation (known compound) in the IR.

Moreover, as a result of the analysis of the obtained compound using HPLC, a plurality of impurity peaks (all of which were 0.03% by weight) were observed as impurities, and the total content of all impurities was 0.12% by weight. Thus, the purity thereof was found to be 99.88% (wherein % by content indicates % with respect to the HPLC area value of a free form of the compound represented by formula (1a)).

Less than 300 ppm ethanol was found as a residual solvent. Furthermore, as a result of the measurement of heavy metal and ignition residue, it was found that almost no inorganic impurities remained.

### (Evaluation Example 1)

The purity of each of the compounds (1a) produced in Reference Example 1 and Examples 1 to 3 was examined using HPLC.

The results are shown in Table 1.

**[Table 1]**

| | | Reference Example 1 | Example 1 | Example 2 | Example 3 |
|---|---|---|---|---|---|
| Appearance | | White crystals | White crystals | White crystals | White crystals |
| Confirmation test (IR) | | Absorption with the same strength at the same wavenumber as that of reference spectrum | Absorption with the same strength at the same wavenumber as that of reference spectrum | Absorption with the same strength at the same wavenumber as that of reference spectrum | Absorption with the same strength at the same wavenumber as that of reference spectrum |
| Impurities | Maximum | 0.03% | 0.03% | 0.03% | 0.03% |
| | Total | 0.16% | 0.13% | 0.12% | 0.12% |

### (Results)

It has found that each compound (1a) produced in Example 1 to 3 comprises impurities, in which the maximum content of any one type of impurity is 0.03% or less and the total content of all impurities is approximately 0.13% or 0.12%, and thus that compound (1a) has a high purity of 99.87% or more (wherein % by content indicates % with respect to the HPLC area value of a free form of the compound represented by formula (1a)).

## Claims

1. High-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate represented by the following formula (1a): 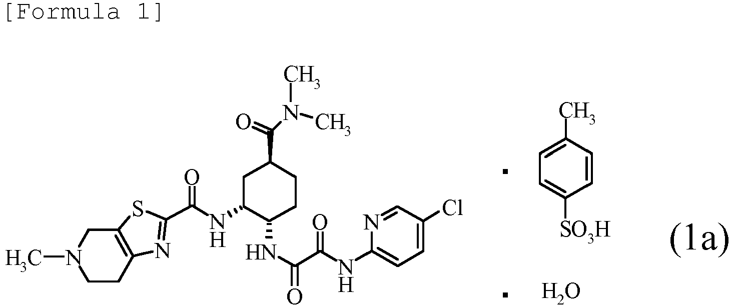 wherein, with regard to the content of impurities, the maximum content of any one type of impurity is 0.03% or less, and the total content of all impurities is 0.13% or less (wherein % by content indicates % with respect to the HPLC area value of a free form of the compound represented by formula (1a)).

2. High-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate represented by the following formula (1a): 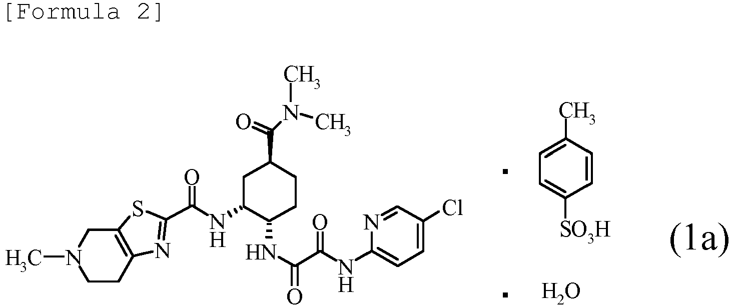
wherein, with regard to the content of impurities, the maximum content of any one type of impurity is 0.03% or less, and the total content of all impurities is 0.13% or less (wherein % by content indicates % with respect to the HPLC area value of a free form of the compound represented by formula (1a)), and wherein
the high-purity crystals are obtained by the following step 1 to step 4:
[Step 1] adding, to N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (compound (1a)), approximately 7 parts by volume of aqueous ethanol [anhydrous ethanol:purified water = 7:3 (V/V)] based on 1 part by weight of compound (1a), and then heating the obtained mixture to an internal temperature of 55°C to 75°C to dissolve compound (1a) in the aqueous ethanol, thereby preparing solution 1;
[Step 2] washing and separating insoluble matter in solution 1, using approximately 1 part by volume of aqueous ethanol, and then using approximately 2 parts by volume of anhydrous ethanol based on 1 part by weight of compound (1a), and thereafter, heating the solution, after completion of the washing and the separation, to an internal temperature of 55°C to 75°C for dissolution, thereby preparing solution 2;
[Step 3] gradually cooling solution 2 to room temperature, adding, to solution 2, approximately 14 parts by volume of anhydrous ethanol based on 1 part by weight of compound (1a), and then cooling the obtained mixture to an internal temperature of 0°C to 15°C to precipitate crystals; and
[Step 4] collecting the crystals, washing the collected crystals with approximately 2 parts by volume of anhydrous ethanol based on 1 part by weight of compound (1a), and then drying the crystals under reduced pressure at a temperature of 50°C or lower.

3. High-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate represented by the following formula (1a): 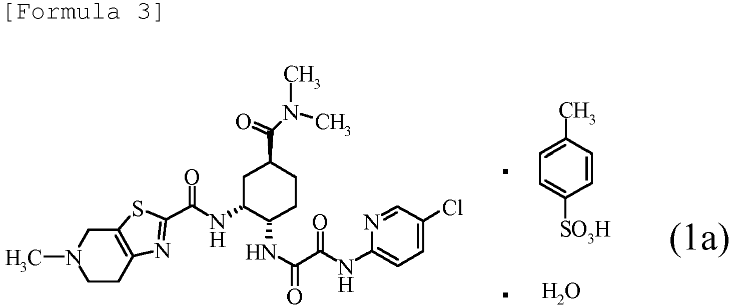
wherein, with regard to the content of impurities, the maximum content of any one type of impurity is 0.03% or less, and the total content of all impurities is 0.12% or less (wherein % by content indicates % with respect to the HPLC area value of a free form of the compound represented by formula (1a)), and wherein
the step 1 to the step 4 of claim 2 are repeated.

4. A method for producing high-purity crystals of N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (1a), wherein the high-purity crystals are obtained by the step 1 to the step 4 of claim 2.

5. Use, as reference standards, of the high-purity crystals N¹-(5-chloropyridin-2-yl)-N²-((1S,2R,4S)-4-[(dimethylamino)carbonyl]-2-{[(5-methyl-4,5,6,7-tetrahydrothiazolo[5,4-c]pyridin-2-yl)carbonyl]amino}cyclohexyl)ethanediamide p-toluenesulfonate monohydrate (1a), which are produced by the production method of claim 4.
